Europäisches Patentamt

European Patent Office

Office européen des brevets

(11) Publication number: **0 427 017 A2**

## EUROPEAN PATENT APPLICATION

(21) Application number: 90119876.2

(51) Int. Cl.5: **G01N 33/531**

(22) Date of filing: **17.10.90**

(30) Priority: **31.10.89 IT 2220689**

(43) Date of publication of application:
**15.05.91 Bulletin 91/20**

(84) Designated Contracting States:
**BE CH DE ES FR GB IT LI LU NL**

(71) Applicant: **S P A SOCIETA' PRODOTTI
ANTIBIOTICI S.p.a.
Via Biella, 8
I-20143 Milan(IT)**

(72) Inventor: **Magnaghi, Sergio
Via Ettore Ponti 30
I-20143 Milan(IT)**
Inventor: **Ferrari, Lorenzo
Via San Simpliciano 6
I-20121 Milan(IT)**
Inventor: **Chiolerio, Francesca
Via Rienza 35
I-22100 Como(IT)**
Inventor: **Malcovati, Massimo
Via San Lanfranco 60
I-27100 Pavia(IT)**
Inventor: **Acerboni, Roberto
Via Pompeo Neri 11
I-20146 Milan(IT)**

(74) Representative: **Luksch, Giorgio, Dr.-Ing. et al
Ing. A. Giambrocono & C. S.r.l. Via Rosolino
Pilo, 19/b
I-20129 Milano(IT)**

(54) **Solid formulations of complex systems like avidin substances-biotinylated marker, useful for bioassays.**

(57) A system useful for immuno-bioassays, consisting of a solid and stable in time formulation comprising a complex formed by avidin, streptavidin or a semi-synthetic derivative thereof, and by a biotinylated marker together with a suitable water-soluble or water-dispersable solid carrier.

EP 0 427 017 A2

The present invention relates to the field of the detection of biological probes, and more specifically to the one of the immuno-enzymatic assays by employing biological probes.

The field concerned by the invention, i .e. the detection of certain macromolecules (defined as "target"), emplies the use of macromolecules (defined as "probe") being able to selectively link the target and to yield a probe-target complex which has to be displaid for the univocal detection.

For the display, the highest affinity between biotin and avidin, or the derivatives thereof, and streptavidin is exploited. Thus the probe is biotinylated, and complexes like avidin substance-biotinylated marker are used as labels, which, by binding target-biotinylated probe complexes, give place to an intense chromatic display of the detected target, owing to the characteristics of the enzyme.

The detection of the target, or rather the procedures for preparing the label provides the mixture of a concentrated solution of avidin, or the like, with a concentrated solution of biotinylated marker, at the moment of the use, and sequentially the dilution of the resulting mixture. Otherwise a concentrated solution of avidin sub stance-biotinylated marker complex already made is diluted at the moment of the use.

Both the above described methods show various drawbacks. As regards the diluted solution there is the problem of the inevitable inaccuracy of the dilution which may compromise the reproducibility of the results, and moreover it has limited stability in time. This latter disadvantage is only partially resolved by employing a concentrated solution that, anyway, can evaporate when the containers get opened, and consequently the reagent concentrations can change, without considering that said solutions, even if usually protected by additives, can be polluted by bacteria or molds anyway.

Said drawbacks yield inevitable waste and high costs that mainly regard the starting materials such as the markers like peroxidase, alkaline phosphatase, and the streptavidin and avidin derivatives which have to be used at a high degree of purity.

U.S. Patent 4,684, 609 discloses a potentiated complex in form of solution, allowing the labelling of probes in histological samples. The reagent consists of avidin and biotinylated macromolecule solutions to be admixed before of the use in preselected ratios, for obtaining a macromolecular complex large enough to address various signal-molecules towards the target, but not so large to pre vent the penetration into the tested tissue sample. From what above said, the drawbacks of such a formulation of the complex are obvious, first of all the stability and the difficulty of having the dosage of reactants precise and stable in time.

The system of the present invention overcomes all the above mentioned problems concerning stability and dosage of the avidin substance-biotinyl ated marker complex, and consequently provides a noticeable decrease of costs and times of usage, as well as a facilitation of the same.

The term "avidin substance" herein means the specific avidin protein as well as its chemically modified forms, also streptavidin. The latter, being extracted from the fermentation broth of Streptomyces avidinii, shows some structural characteristics different from the ones of avidin, but its behaviour concerning the binding with biotin is completely superimposable on the one of such protein.

The term "biotinylated marker" herein means any enzyme commonly employed as detector in bioassays, such as peroxidase, alkaline phosphatase, B-galactosidase, glucosoxi dase, etc., also other no-protein substances that provide signal, e.g. fluorescence, such as fluorescein and rhodamine. Such markers are covalently binded to biotin in a molar ratio biotin:enzyme > 1.

The system of the present invention is economically advantageous and handy, and renresents a noticeable improvement in comparison with the methods known hitherto. Such system consists of a multi-dose formulation made by an analitically inert water-soluble or water-dispersable support , suitably associated to a calibrated amount of preformed complex like avidin substance-biotinylated marker, being such formulation in form of tablets readily soluble in water or in an aqueous medium employed for the analysis, such as to permit of obtaining a solution just diluted at the concentration of employment; the tablets in view of the kind of support and formulations remain unalterate at least until one year after the manifacture of the oroduct.

The excipient preferably used in the present invention is a disaccharide, e.g. lactose, though mono- and polysaccharides can be employed, so as different kind of substances such as polyalcohols, amides, etc., provided that they are water-soluble or anyway readily water-dispersable, inert and consistent with the macromolecules forming the active ingredient. As consistency, it is meant the determinative factor for the conservation of the complex activity for a period not shorter than one year.

The avidin substance-biotinylated marker active complex is made by a mixture of aggregates having molecular weight in the range between 200.000 and also behind 1 ,000,000 daltons. In the active complex each molecule of biotinylated enzyme binds at least two molecules of avidin substance so as to form a "cluster" structure that sequentially interacts with the biotinyl ated probe-target system by the still free avidine sites. The molar ratio avidin: :biotinylated enzyme into a tablet of the present invention ranges between 1 10 and 2: 1, and is preferably of about 1: 1. It has to be underlined that the ratio avidin substance-biotinylated marker has its limit to 2:1 because beyond such value the specificity of the complex for the biotinylated probe is last, so altering the result of the detection.

The amount of the avidin substance-biotinylated marker complex to incorporate in the eccipient is calculated on the basis of the chromatic responses obtained through dosage in ELISA system, which is described below. Such amount is at least of $1\mu g/20mg$ of tablet, preferably of $1\mu g/2mg$ of tablet. In the final formulation, a small excess of complex in comparison with the minimum found amout is acceptable without prejudice, but it is not essential

As one of the main aspects of the present invention concerns the noticeable stability of the preparation in the described formulation and such stability significan tly depends on the instant humidity degree, some operative rules have to be scrupulously followed during the packing step and in the step therein before. Before distributing into the squibs, that are previously half-filled with activated silica gel, the tablets have to be deeply dried in a phosphoric anhydride vacuum dryer for at least 24 hours. The distribution has to take place in a dehumidified environment, and the squibs must have hermetic seal. Moreover the direct light must be avoided as much as possible.

For better explaining the embodiments of the present invention, examples are below provided, being not be intended as limiting the invention.

## EXAMPLE 1

Preparation of a modified avidin

1 g (0.015 mmole) of native avidin having a high degree of purity is dissolved in 100 ml of 0.05 M tris(hydroxymethyl )aminomethane buffer (TRIS), ptt 8.0. To this stirred solution, 0.18 ml (1.9 mmole) of acetic anhydride is added. The pH is adjusted to 8.0 by means of a solution of 20% sodium hydrate, and the solution is maintained under stirring for further 20 min, then it is dialyzed against 4x2 1 changes of bidistilled water, at a temperature of $4^{\circ}$ C.

To so obtained solution, containing about 0.9 g of a cetyl-avidin, is freeze-dried and stored as such in strictly anhydrous environment.

In a similar way other modified avidins such as succinyl-avidin and phthalyl-avidin are prepared by employing succinic and phthalic anhydride, respectively, in the place of acetic anhydride, in suitable ratios.

## EXAMPLE 2

Preparation of a biotinylated enzyme

100 mg (about 25,000 U) of horseradish peroxidase are dissolved in 8 ml of a solution of 0,1 M sodium bicarbonate, pH 8.0. Then a solution of 80 mg of -caproylamido-biotin-N-hydroxysuccinimide ester in 2 ml of DMF is added. The reaction mixture is put under stirring for 1 hour at room temperature and is then dialyzed for 60 hours against 4-5 x 800 ml changes of PBS buffer (phosphate buffered saline composition in 1 1: $Na_2HPO_4 = 0.916$ g; $KCl = 0.2$ g; $NaCl = 8$ g; $KH_2PO_4 = 0.2$ g).

100 mg of AH-biotinylated-peroxidase are obtained in about 16 ml of 0.15 M PBS buffer solution, pk 7.5. After an assay for verifying the enzymatic activity of the biotin and protein content, and the subsequent optional dilution, said solution is used as such to prepare complexes with freeze-dried avidin.

## EXAMPLE 3

Preparation of avidin-biotinylated enzyme complexes

a) Solution A consists of 60 mg of acetyl-avidin with a specific activity of 12.1 U/mg, dissolved in 10 ml of bidistilled water containing 10 mg of phenol and 150 mg of BSA (bovine serum albumin).

Solution B comprises AM-biotinylated-peroxidase at an original concentration of 5 mg/ml (about 1,200 U/ml of peroxidase, in 0.15 M PBS (pM 7.5), the enzyme being stabilized with 0.02% of benzethonium chloride. 10 ml of solution B are slowly added to solution A under moderate stirring while maintaining the temperature at about $4^{\circ}$ C. At the end of the adding and after some further minutes of stirring, the resulting solution of AB complex is filtered under sterile conditions through a $0,2\mu m$ filter, and then vigorously stirred and freeze-dried. The freeze-dried has to be stored under vacuum.

b) Solution A is prepared by accurately dissolving 23.9 mg of streptavidin with an activity of 15.2 U/mg, in 5 ml of 0.15 M PBS, pM 7.5.

As solution B, a solution of AM-biotinytated-peroxidase in 0.15 MBS, pM 7.5, is employed, stabilized with of 0.02% benzethonium chloride, at an original concentration of 5 mg/ml (about 1,200 U/ml ) of peroxidase. The AB complex solution is prepared as described in at, but using 5 ml of solution B.

c) Solution A is prepared by dissolving 2% p/v of BSA, 11% p/v of NaCl , 0.925% of succinyl avidin (activity = 7.85 U/mg) in 10 ml of bidistilled water. The pM is adjusted to 7.5 by addition of bibasic sodium phosphate.
Solution B is prepared as in a), and following such procedure the AB complex solution is prepared using 10 ml of solution A and 10 ml of solution B.

d) Solution A consists of 95.5 mg of phthalyl-avidin dissolved in 10 ml of 0.15 M PBS (pM 7.5) containing 0.1% of benzethonium chloride, and carefully stirred for preparing solution B, 155 mg of previously freeze-dried AM-biotinylated-peroxidase, corresponding to 50 mg of peroxidase, are dissolved in 10 ml of 0.15 M PBS (pM 7.5).
Solution B is very rapidly added to solution A, and the mixture is stirred for no longer than 15 seconds. Then freeze-drying is effected as in a).

e) 30 mg of streptavidin with an activity of 15.2 U/mg are dissolved in 10 ml of bidistilled water containing 10 mg of phenol and 150 mg of BSA (solution A). Solution B consists of 30 mg of biotinylated alkaline phosphatase dissolved in 10 ml of a buffered solution of 0.4 mg/ml of sodium azide, 0.1 mM of zinc chloride, 3 M of sodium chloride, 30 mM of triethanolamine, 1mm of magnesium chloride.
For obtaining the AB complex solution, 10 ml of solution B and 10 ml of solution A are used admixed at a temperature of 406 under moderate stirring. The AB complex solution is freeze-dried according to common techniques.

## EXAMPLE 4

Determination of the amount of active ingredient to incorporate in the excipient.

Freeze-dried AB complex obtained in Example 3 is weighed, and an aliquot corresponding to 0.5 ml of the original solution is taken off. This aliquot is dissolved in 0.5 ml of bidistilled water under moderate stirring. By means of micropipet, scalar volumes of this solution (20, 23, 26, 29, 32, 35, 38 and 41 1) are added to a group of eight 10 ml vials, each containing 150 mg of excipient consisting of 5 g of L-leucine base homogeneously blended with 95 g of dry lactose (the excipient mixture was been previously dried under vacuum in a phosphoric anhydride drier), and this solution is contacted therewith for 10-15 minutes. Afterwards, 6 ml of 0.15 M PBS containing 0.1% of Tween 20, are added to each vial,shortly shaken, left to stand for further 10-15 minutes, and then shortly shaken again. The so obtained solutions are subjected to a dosage in ELISA system. By this way the minimum amount to be incorporate into the excipient is established on the basis of the chromatic responses obtained.

## EXAMPLE 5

ABC dosage in ELISA System

For this test 96-wells microtitration plates are used.

a) Preparation of the solid layer.

Each well of the microplate is coated with 100 μl of a solution of biotinylated BSA, diluted to a concentration of 1 μg/ml in 50 mM carbonate-bicarbonate buffer, pM 9.5. The wells excluded from the coating are emploied as "blank" for the spectrophotometric resetting. The plate is O/N incubated at room temperature, then washed 3 times with the washing buffer (PBS + 0.05% of Tween 20).
Each well of the microplate is overcoated with 300 μl of PBS containing 0.5% Tween 20 and 10% of bovine serum. The plate is incubated for 4 hours at room temperature, then washed 3 times with the above washing buffer.

b) Execution of the text.

Samples and standard kits are diluted at the use concentration in PBS + 0.5% Tween 20. 100 μl of each solution, in quadruplicate, are placed into wells of the microplates, then incubated 30 minutes at room temperature and accurately washed 5 times with the washing buffer as in a).
In each well 100 μl of chromogen substrate solution for peroxidase are placed, containing:
- 1 ml of 40 mM 2,2′-azino-bis(3-ethylbenzthiazoline-6-sulfonic acid) (ABTS) in bidistilled water;
- 20ml of 0.1 M sodium acetate and 0.05 M of monobasic sodium phosphate buffer;
- 0.2 ml of 0.25 M $H_2O_2$, in bidistilled water.
The spectrophotometer is reset at 405 nm against a "blank" sub strat e solution. The assorbance values of the single well are read after about 5-10 minutes. The samples are evaluated on the basis of their chromatic response as compared with standards.

## EXAMPLE 6

The dosage of Example 5 has been effected according to the procedure of said Example but employing 100$\mu$l of substrate chromogen solution composed as follows:
- 0,5 ml of 40 mM 2,2'-azino-bis(3-ethylbenzthiazoline-6-sulfonic acid) (ABTS) in bidistilled water;
- 10 ml of 0.1 M sodium acetate and 0.05 of monobasic sodium phosphate buffer;
- 0.1 ml of 0.25 M $H_2O_2$ in bidistilled water.

## EXAMPLE 7

Preparation of mini- tablets

a) The freeze-dried AB complex obtained in Example 3-a) is blended, in anhydrous environment, with the excipient made of 5 a of L-leucine base and 95 g of dry lactose, in a weight ratio of 1 1100 of excipient equivalent to 45 $\mu$g/50 mg tablet. The mini-tablets are prepared according to techniques and by means of equipments known in the art.

b) 50 g of L-leucine are mixed with 950 g of TABFINE 097 (HS)[R] (96-98% dextrose from Edward Mendell ). The preparation of the tablets follows the same procedure described in a), incorporating the AB active ingredient obtained in Example 3-b), in an amount of 45 g/50 mg tablet.

c) Following the same procedure described in a) and using the excipient in b), the AB complex prepared in Example 3-c) is used in an amount of 57$\mu$/50 mg of excipient.

d) Following the procedure of and using the same excipient of a), the active ingredient prepared according to Example 3-d) is incorporated in an amount of 50 g/50 mg of excipient.

e) 5 g of base L-leucine are mixed with 95 g of an hydrous lactose (Tablettose[R]) (Meggle Milkindustrie). The tablets are prepared according to the procedure described in a), incorporating the active ingredient obtained in Example 3-e) into the excipient in an amount of 48 $\mu$g/50 mg of excipient.

f) 10 $\mu$l of an AB complex solution as prepared in Example 3-a), are disposed by micro-dispenser on the surface of a 50 mg tablet consisting in anhydrous lactose containing 5% of L-leucine. The so treated tablets are placed in a phosphoric anhydride drier and dried therein for 24 hours under vacuum.

## EXAMPLE 8

320 mg of acetyl-avidin (12 U/mg) are homogenized with 10 g of anhydrous lactose. Separately, 780 mg of AM-biotinylated peroxidase (equivalent to 270 mg of peroxidase) are homogenized with 10 g of anhydrous lactose.

The two powders, previously passed through a fine sieve, are admixed in equal parts in a suitable mixer. Then 20 g of such mixture are intimately incorporated in the excipient made of 455 g of anhydrous lactose and 25 g of L-leucine base. After further sieving, the product is placed in a phosphoric anhydride drier, under vacuum for 24 hours at room temperature.

The compression is thereafter effected according to common ways for obtaining 50 mg tablets.

## EXAMPLE 9

Way of employment of the mini-tablets

A 50 mg mini-tablets is placed in a small vial or test tube containing 2 ml of 0.15 M PBS buffer at pM 7.5, added with 0.1% of Tween 20. The mixture is shortly shaken and then is left to stand for 10-15 minutes. Shake again. The obtained solution is directly used as labelling system for biotinytated probes.

## Claims

1. Formulation for bioassays consisting of a solid and stable in time formulation, comprising a complex made of a substance selected from the group consisting of streptavidin, avidin and semi-sinthetic derivatives thereof, and a biotinylated marker, supported by an analitically inert watersoluble or water-dispersable solid carrier.

2. The formulation according to claim 1, wherein the complex is in an amount of at least 1 $\mu$g/20 mg of solid carrier.

3. The formulation according to claim 2, wherein the complex is in an amount of 1 $\mu$g/ 2 mg of solid carrier.

4. The formulation according to the previous claims, wherein the ratio avidin substance:biotinylated marker is in the range between 1:10 and 2:1 in the solid formulation.

5. The formulation according to claim 4, wherein the ratio avidin substance:biotinylated marker is of about 1:1 in the solid formulation.

6. The formulation according to the previous claims, wherein the marker is an agent being able to yield colour formation in a chromogenic substrate.

7. The formulation according to the previous claims, wherein the marker is an agent being able to give visually signal or strumental ly detectable radiations.

8. A process for preparing the formulation according to the preceeding claims characterized in that

i) a solution of biotinylated marker is admixed with a solution of an avidin substance selected from the group consisting of streptavidin, avidin and semi-sinthetic derivatives thereof, in a ratio between 10:1 and 1 2, the resulting mixture is stirred while maintaining the temperature at $0°$ - $10°C$ to obtain a complex that is then freeze-dried and blended with at least one anal itical ly inert water soluble or water-dispersable solid carrier in a ratio of from 1 $\mu$g/20 mg of solid carrier to 1$\mu$g/2mg of solid carrier; or

ii ) a solution of an avidin substance and a solution of biotinylated marker are separately blended with an analitically inert watersoluble or water-dispersable solid carrier, and the two powders are then admixed and added with a further amount of at least one of the above solid carrier, to so-obtained powder being stored and compressed under anhydrous conditions.